(19) Europäisches Patentamt
European Patent Office
Office européen des brevets

(11) Publication number: 0 411 900 A2

(12) # EUROPEAN PATENT APPLICATION

(21) Application number: 90308412.7

(51) Int. Cl.[5]: A61K 31/415

(22) Date of filing: 31.07.90

The title of the invention has been amended (Guidelines for Examination in the EPO, A-III, 7.3).

(30) Priority: 01.08.89 GB 8917556

(43) Date of publication of application: 06.02.91 Bulletin 91/06

(84) Designated Contracting States: AT BE CH DE DK ES FR GB GR IT LI LU NL SE

(71) Applicant: GLAXO GROUP LIMITED
Clarges House 6-12 Clarges Street
London W1Y 8DH(GB)

(72) Inventor: Cooper, Steven John, University of Birmingham
Department of Psychology, Edgbaston
Birmingham, B15 2TT(GB)

(74) Representative: Marchant, James Ian et al
Elkington and Fife Beacon House 113 Kingsway
London WC2B 6PP(GB)

(54) **Treatment of conditions involving excessive eating.**

(57) The invention relates to the use of ondansetron or a physiologically acceptable salt or solvate thereof, particularly ondansetron hydrochloride dihydrate, in the treatment of bulimia or a related disorder.

EP 0 411 900 A2

# MEDICAMENTS

This invention relates to a new medical use for a heterocyclic compound and pharmaceutical compositions containing it. In particular it relates to the use of 1,2,3,9-tetrahydro-9-methyl-3-[(2-methyl-1 H -imidazol-1-yl)methyl]-4 H -carbazol-4-one and the physiologically acceptable salts and solvates thereof in the treatment of a condition involving excessive eating.

French Patent Specification No. 2625678 discloses the use of various quinuclidine benzamides and thiobenzamides, together with their N-oxides, hydrates and salts in the treatment of increased weight and obesity of various causes. The use of 4-amino-5-chloro-2-methoxy-N-(quinuclidin-3-yl)benzamide (which has the approved name zacopride), a compound which is known to be an antagonist of 5-hydroxytryptamine (5-HT) at $5\text{-}HT_3$ receptors, is specifically described.

1,2,3,9-Tetrahydro-9-methyl-3-[(2-methyl-1 H -imidazol-1-yl)methyl]-4 H -carbazol-4-one, which has the approved name ondansetron, may be represented by the formula (I):

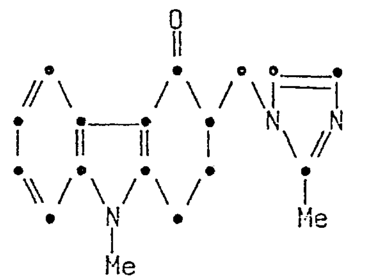

(I)

and is described and claimed in UK Patent Specification No. 2153821B.

Suitable physiologically acceptable salts of ondansetron include acid addition salts formed with organic or inorganic acids for example, hydrochlorides, hydrobromides, sulphates, phosphates, citrates, fumarates and maleates. The solvates may, for example, be hydrates.

Ondansetron is described in the aforementioned specification as a potent and selective antagonist of 5-hydroxytryptamine (5-HT) at 'neuronal' 5-HT receptors of the type located on terminals of primary afferent nerves. Receptors of this type are now designated as $5\text{-}HT_3$ receptors and are also present in the central nervous system. 5-HT occurs widely in the neuronal pathways in the central nervous system and disturbance of these 5HT containing pathways is known to alter behavioural syndromes such as mood, psychomotor activity, appetite and memory.

Ondansetron is described in UK2153821B as being of use in the treatment of a human or animal subject suffering from a condition caused by a disturbance of neuronal 5-HT function, for example in the treatment of a human subject suffering from migraine pain or a psychotic disorder such as schizophrenia. It is also stated that the compound may be useful in the treatment of conditions such as anxiety, obesity and mania.

European Patent Specification No. 226266 describes the use of ondansetron in promoting gastric emptying, and as being of use in the treatment of conditions which may be relieved by the promotion of gastric emptying. Such conditions include gastric stasis and symptoms of gastrointestinal dysfunction such as dyspepsia, reflux oesophagitis, peptic ulcer and flatulence. EP-A-226266 further describes ondansetron as an anti-emetic, and as being useful in the treatment or prevention of nausea and vomiting. The use of the compound for the treatment of emesis is also described in published European Patent Specification No. 201165, which specification additionally refers to the treatment of irritable bowel syndrome.

According to published European Patent Specifications Nos. 275668, 276559 and 278161, ondansetron may also be used in the treatment of dementia and other cognitive disorders, depression, and dependency on drugs or substances of abuse.

It has now been found that ondansetron is useful for the treatment of bulimia. Bulimia (and more specifically bulimia nervosa) is a condition in which there is an abnormal desire for food, particularly rich, sweet and highly palatable foods, which leads to over-indulgence and bouts of binge eating. This is combined with an overconcern for body size, such that excessive eating is often followed by purging and/or vomiting. Ondansetron is also useful for the treatment of related disorders such as seasonal affective disorder (SAD).

The ability of ondansetron to suppress the intake of palatable foods has been demonstrated in rats.

Thus in rats familiarised with eating an enriched sweetened mash and injected intraperitoneally with ondansetron hydrochloride dihydrate in doses of either 3, 10 or 30$\mu$g $kg^{-1}$, significant reductions in food intake were found at each dose level. Thus, for example, a dose of 10$\mu$g $kg^{-1}$ produced a 42% reduction in intake.

Accordingly the invention provides a method of treatment of a subject suffering from bulimia or a related disorder, which comprises administering to the subject an effective amount of ondansetron or a physiologically acceptable salt or solvate thereof.

References in this specification to treatment include prophylactic treatment as well as the acute alleviation of symptoms.

A preferred form of ondansetron is the hydrochloride, particularly in a hydrated form (e.g. the dihydrate).

In a further aspect, the invention provides a pharmaceutical composition which comprises an effective amount of ondansetron or a physiologically acceptable salt or solvate (e.g. hydrate) thereof, for use in medicine, particularly human medicine, for the treatment of bulimia or a related disorder.

In a yet further aspect, the invention provides for the use of ondansetron or a physiologically acceptable salt or solvate thereof, for the manufacture of a medicament for the treatment of bulimia or a related disorder.

Pharmaceutical compositions for use in accordance with the present invention may be formulated in conventional manner using one or more physiologically acceptable carriers or excipients.

Thus ondansetron and its physiologically acceptable salts and solvates may be formulated for oral, buccal, parenteral, rectal or transdermal administration or in a form suitable for administration by inhalation or insufflation (either through the mouth or the nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g lecithin or acacia); non-aqueous vehicles (e.g. almond oil, oily eaters, ethyl alcohol or fractionated vegetable oils); and preservatives (e.g. methyl or propyl-p -hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

Ondansetron may be formulated for parenteral administration by injection e.g. by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

Ondansetron may also be formulated in rectal compositions such as suppositories or retention enemas, e.g. containing conventional suppository bases such as cocoa butter or other glycerides.

In addition to the formulations described previously, the compound may also be formulated as a depot preparation. Such long acting formulations may be administered by implantation (for example subcutaneously, transcutaneously or intramuscularly) or by intramuscular injection. Thus, for example, ondansetron may be formulated with suitable polymeric or hydrophobic materials (for example as an emulsion in an acceptable oil) or ion exchange resins, or as sparingly soluble derivatives, for example, as a sparingly soluble salt.

A proposed dose of ondansetron for use according to the invention for administration to a subject (of approximately 70kg body weight) is 0.05 to 20mg, preferably 0.1 to 10mg of the active ingredient per unit dose, expressed as the weight of free base. The unit dose may be administered, for example, 1 to 4 times per day. The dose will depend on the route of administration. It will be appreciated that it may be necessary to make routine variations to the dosage depending on the age and weight of the patient as well as the

severity of the condition to be treated.

Ondansetron may be prepared by the processes described in UK Patent Specification No. 2153821B, and the following examples illustrate its preparation and salt formation. Temperatures are in °C.

Example 1

1,2,3,9-Tetrahydro-9-methyl-3-[(2-methyl-1H-imidazol-1-yl)methyl]-4H-carbazol-4-one

A solution of 3-[(dimethylamino)methyl]-1,2,3,9-tetrahydro-9-methyl-4H-carbazol-4-one hydrochloride (1.7g) in water (17ml) was treated with 2-methylimidazole (1.4g) and then heated under reflux for 20h. The cooled mixture was filtered and the residue washed with water (3x15ml) to give a product (1.7g) m.p. 221-221.5°. This material was recrystallised from methanol to give the title compound (1.4g) m.p. 231-232°.

Example 2

1,2,3,9-Tetrahydro-9-methyl-3-[(2-methyl-1H-imidazol-1-yl)methyl]-4H-carbazol-4-one hydrochloride dihydrate

1,2,3,9-Tetrahydro-9-methyl-3-[(2-methyl-1H-imidazol-1-yl)methyl]-4H-carbazol-4-one (18.3g) in a hot mixture of isopropanol (90ml) and water (18.3ml) was treated with concentrated hydrochloric acid (6.25ml). The hot mixture was filtered and the filtrate diluted with isopropanol (90ml) and stirred at room temperature for 17h, cooled to 2° and the solid filtered off (21.6g). A sample (6g) was recrystallized from a mixture of water (6ml) and isopropanol (10ml) to give the title compound as a white crystalline solid (6g) m.p. 178.5-179.5°.

| | | | |
|---|---|---|---|
| Analysis Found: | C,59.45; | H,6.45; | N,11.5. |
| $C_{18}H_{19}N_3O.HCl.2H_2O$ requires | C,59.1; | H,6.6; | N,11.5%. |
| Water assay Found: | 10.23% | | |
| $C_{18}H_{19}N_3O.HCl.2H_2O$ requires | 9.85% | | |

The following examples illustrate pharmaceutical formulations for use according to the invention, containing ondansetron hydrochloride dihydrate as the active ingredient (1.25g of the hydrochloride dihydrate contains 1.00g of the free base).

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression Tablet

| | mg/tablet |
|---|---|
| (i) Active Ingredient | 4.688 |
| Calcium Hydrogen Phosphate BP* | 83.06 |
| Croscarmellose Sodium NF | 1.8 |
| Magnesium Stearate BP | 0.45 |
| Compression weight | 90.0 |

* of a grade suitable for direct compression.

The active ingredient is passed through a 60 mesh sieve, blended with the calcium hydrogen phosphate, croscarmellose sodium and magnesium stearate. The resultant mix is compressed into tablets using a Manesty F3 tablet machine fitted with 5.5mm, flat bevelled edge punches.

| | mg/tablet |
|---|---|
| (ii) Active Ingredient | 0.31 |
| Anhydrous Lactose USNF | 131.99 |
| Pregelatinised Starch USNF | 7.0 |
| Magnesium Stearate BP | 0.7 |
| Compression weight | 140.0 |

The active ingredient is passed through a 60 mesh sieve, and blended with the lactose, pregelatinised starch and magnesium stearate. The resultant mix is compressed into tablets using a Manesty F3 tablet machine fitted with 7.5mm normal concave punches.

## INJECTION

The injection may be administered by the intravenous or subcutaneous route.

| | µg/ml |
|---|---|
| Active Ingredient | 800 |
| Dilute Hydrochloric Acid BP | to pH 3.5 |
| Sodium Chloride Injection BP | to 1ml |

The active ingredient is dissolved in a suitable volume of Sodium Chloride Injection BP, the pH of the resultant solution is adjusted to pH3.5 with dilute hydrochloric acid BP then the solution is made to volume with sodium chloride injection BP and thoroughly mixed. The solution is filled into Type 1 clear glass 5ml ampoules which are sealed under a headspace of air, by fusion of the glass then sterilised by autoclaving at $120^0$ for not less than 15 minutes.

## SUPPOSITORY

| | |
|---|---|
| Active Ingredient | 5.0mg |
| * Witepsol H15 to | 1.0g |

* Witepsol H15 is a proprietary grade of Adeps Solidus Ph. Eur.

A suspension of the active ingredient is prepared in the molten Witepsol and filled, using suitable machinery, into 1g size suppository moulds.

## Claims

1. The use of ondansetron or a physiologically acceptable salt or solvate thereof for the manufacture of a medicament for the treatment of bulimia or a related disorder.
2. Use according to Claim 1 for the manufacture of a medicament adapted for oral, buccal, parenteral, rectal or transdermal administration or in a form adapted for administration by inhalation or insufflation.
3. Use according to Claim 1 or 2 for the manufacture of a medicament in unit dose form containing from 0.05 to 20 mg of active ingredient per unit dose expressed as the weight of free base.
4. Use according to Claim 3 wherein the amount of active ingredient per unit dose is from 0.1 to 10 mg.
5. Use according to any of Claims 1 to 4 wherein the ondansetron is used in the form of a hydrochloride salt.
6. Use according to Claim 5 wherein the ondansetron is used in the form of ondansetron hydrochloride dihydrate.